(19) Europäisches Patentamt / European Patent Office / Office européen des brevets

(11) **EP 4 764 482 A1**

(12) **EUROPEAN PATENT APPLICATION**

(43) Date of publication:
24.06.2026 Bulletin 2026/26

(21) Application number: 25212091.0

(22) Date of filing: **29.10.2025**

(51) International Patent Classification (IPC):
**G01N 27/12** *(2006.01)* **G01N 33/00** *(2006.01)*

(52) Cooperative Patent Classification (CPC):
**G01N 27/127; G01N 33/0031**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **23.12.2024 KR 20240193844**

(71) Applicant: **Daegu Gyeongbuk Institute of Science and Technology**
**Dalseong-gun, Daegu 42988 (KR)**

(72) Inventors:
• KWON, Hyuk-Jun
42988 Daegu (KR)
• LIM, Hyeong Tae
43014 Daegu (KR)
• KWON, Hyeok Jin
28340 Cheongju-si (KR)

(74) Representative: **Prüfer & Partner mbB**
**Patentanwälte · Rechtsanwälte**
**Sohnckestraße 12**
**81479 München (DE)**

(54) **ELECTRONIC NOSE INCLUDING LASER-INDUCED GRAPHENE EMBEDDED WITH GAS-ACTIVE PARTICLES AND METHOD OF MANUFACTURING THE SAME**

(57)    An electronic nose, comprising: a substrate including a carbon precursor and a gas-active material precursor; and an olfactory sensor array formed on the substrate and comprising a plurality of olfactory sensors, wherein the plurality of olfactory sensors comprise a laser-induced graphene layer formed by irradiating the substrate with a laser and gas-active particles embedded in the laser-induced graphene layer, and wherein the plurality of olfactory sensors are formed by respectively irradiating lasers having different parameters such that the plurality of olfactory sensors respond to different types of gaseous substances.

FIG. 1

## Description

### CROSS-REFERENCE TO RELATED APPLICATION

This application claims priority from and the benefit of Korean Patent Application No. 10-2024-0193844 filed on December 23, 2024, in the Korean Intellectual Property Office, the disclosure of which is incorporated herein by reference.

### BACKGROUND OF THE DISCLOSURE

### Field of the Disclosure

The present invention relates to an electronic nose and a method of manufacturing the same, and more particularly, to an electronic nose comprising a plurality of olfactory sensors configured to respond to different types of gaseous substances, the plurality of olfactory sensors including laser-induced graphene embedded with gas-active particles formed by a simple process of irradiating lasers having different parameters, and to a method of manufacturing the same.

### Description of the Related Art

The digitization of human senses has promoted innovation across various technologies and has significantly transformed daily life. However, the digitization of the sense of smell remains a challenging field.

An electronic nose is a device inspired by the human nose that detects and identifies odors upon exposure to odor molecules. Such an electronic nose provides enormous potential in various fields including environmental monitoring, food safety, healthcare, and the fragrance industry.

Specifically, it is known that olfactory receptors in the human body do not bind to odor molecules in a one-to-one manner. Instead, olfactory receptors transmit information about odors through a combination coding process in which odor molecules bind to multiple types of olfactory receptors. Through this mechanism, humans, with about 400 types of olfactory neurons, are known to be capable of distinguishing approximately one trillion different odors. Accordingly, the development of electronic nose technology aims to mimic the same functions and structures of the human olfactory system in order to achieve digitization of the sense of smell.

Conventionally, techniques for fabricating electronic noses that mimic the human olfactory system have included: (i) electronic nose sensor array fabrication techniques by mounting individual bulk sensors, (ii) electronic nose fabrication techniques through deposition of metal oxides, (iii) electronic nose fabrication techniques based on carbon compounds, and (iv) single gas sensor fabrication techniques based on laser-induced graphene.

In particular, electronic nose sensor array fabrication through the mounting of individual sensors is known to simulate the mechanism of olfaction by arranging individual gas sensors with different reactivities in parallel, and predicting gas species by analyzing the response patterns obtained from each sensor.

Further, electronic nose fabrication through deposition of metal oxides is known to integrate a metal oxide sensor array onto a single substrate using semiconductor patterning (exposure-deposition) processes, instead of assembling an electronic nose from individual gas sensors, thereby being advantageous for miniaturization and sensor diversification.

In addition, electronic nose fabrication based on carbon compounds is known as a technology for fabricating gas sensor arrays based on highly reactive carbon compounds (such as reduced graphene oxide (rGO)) in order to operate the sensors at room temperature.

In particular, the single gas sensor fabrication technique based on laser-induced graphene has been widely studied due to the large surface area and abundant functional groups of laser-induced graphene, making it advantageous for the development of single gas sensors targeting individual gas species. It is also known that the laser-induced graphene-based technique generally utilizes hybrid materials with functional catalysts (such as VOx, MOFs, $MoS_2$), rather than employing laser-induced graphene alone.

However, electronic nose sensor array fabrication using individual sensors is limited in miniaturization and sensor diversification because it comprises an assembly of individual gas sensors with different reactivities, each of which is bulky.

In particular, electronic nose fabrication using deposition of metal oxides requires complex exposure processes and vacuum deposition steps for patterning, which not only consumes substantial time and cost but also requires high-temperature operation (>150 °C) due to the operating mechanism of metal oxide gas sensors.

In contrast, electronic nose fabrication using carbon compounds can operate at room temperature and allows relatively free reactivity tuning. In such fabrication of electronic noses using carbon compound-based gas sensors (such as rGO), particles synthesized by solvothermal synthesis or similar processes are delivered through ex-situ techniques such as drop-casting. However, the ex-situ approach is known to have limitations due to agglomeration phenomena and manual fabrication issues, resulting in large variations between sensors and requiring significant time, thus limiting mass production.

Moreover, the single gas sensor fabrication technique based on laser-induced graphene has primarily been studied only as a single channel, and has not yet been applied to fabrication of an electronic nose in the form of an array for classifying multiple types of gases.

[Prior Art Documents]

[Patent Documents]

(Patent Document 1) Korean Patent No. 10-1852074 (Publication Date: April 25, 2018)

## SUMMARY

It is an object of the present invention to provide an electronic nose including an olfactory sensor array based on a laser-induced graphene sensor fabrication technique, the olfactory sensor array being configured to respond to different types of gaseous substances.

It is another object of the present invention to provide a method of manufacturing an electronic nose capable of performing substrate fabrication and sensor array formation in-situ, thereby enabling fabrication of the olfactory sensor array within a short time. In one embodiment, an electronic nose comprising: a substrate comprising a carbon precursor and a gas-active material precursor; and an olfactory sensor array comprising a plurality of olfactory sensors formed by respectively irradiating the substrate with lasers having different parameters, wherein each of the olfactory sensors comprises a laser-induced graphene layer formed from the carbon precursor and gas-active particles formed from the gas-active material precursor and embedded in the laser-induced graphene layer by laser irradiation with a single parameter, wherein the plurality of olfactory sensors generates a distinct response pattern for each type of gaseous substance, enabling their discrimination.

In one embodiment, a method of manufacturing an electronic nose, comprising: preparing a mixed solution comprising a gas-active material precursor and a carbon precursor; forming a substrate using the mixed solution; and generating an olfactory sensor array comprising a plurality of olfactory sensors by respectively irradiating the substrate with lasers having different parameters, wherein each olfactory sensor comprises a laser-induced graphene layer formed from the carbon precursor and gas-active particles formed from the gas-active material precursor and embedded in the laser-induced graphene layer by laser irradiation with a single parameter, and wherein the plurality of olfactory sensors generates a distinct response pattern for each type of gaseous substance, enabling their discrimination.

## BRIEF DESCRIPTION OF THE FIGURES

Embodiments will be described in more detail with regard to the figures, wherein like reference numerals refer to like parts throughout the various figures unless otherwise specified, and wherein:

FIG. 1 is a configuration diagram of an electronic nose (1) including an olfactory sensor array (20) comprising a porous laser-induced graphene layer (21) in which gas-active particles (23) are embedded, according to an embodiment.

FIG. 2 is a flowchart illustrating a method of manufacturing an electronic nose according to an embodiment.

FIG. 3 shows analysis results of characteristics of a CeLIG sensor formed by the method according to an embodiment.

FIG. 4 shows analysis results of chemical and physical diversity of a CeLIG sensor array formed by the method according to an embodiment.

FIG. 5 shows response data of the CeLIG sensor array to different odor substances, the CeLIG sensor array being formed by the method according to an embodiment.

FIG. 6 illustrates a machine learning algorithm-based odor classification training process of the CeLIG sensor array according to an embodiment.

FIG. 7 shows odor prediction data and performance comparison results based on a machine learning algorithm of the CeLIG sensor array according to an embodiment.

FIG. 8 shows stress analysis results and flexibility test results of the CeLIG sensor array according to an embodiment.

## DETAILED DESCRIPTION OF THE DISCLOSURE

Specific structural or functional descriptions of embodiments according to the concept of the present invention disclosed herein are merely illustrated for the purpose of explaining the embodiments according to the concept of the present invention. The embodiments according to the concept of the present invention may be implemented in various forms, and are not limited to the embodiments described in this specification.

The embodiments according to the concept of the present invention may be modified in various ways and may take various forms, and thus the embodiments are illustrated in the drawings and described in detail herein. However, this is not intended to limit the embodiments to specific disclosed forms, but is intended to include modifications, equivalents, or substitutions that fall within the spirit and scope of the invention.

Terms such as "first" or "second" may be used to describe various elements, but the elements should not be limited by such terms. The terms are used only for the purpose of distinguishing one element from another. For example, a first element may be referred to as a second element without departing from the scope of the invention, and similarly, a second element may also be referred to as a first element.

When an element is referred to as being "connected to" or "coupled to" another element, it may be directly connected or coupled to the other element, or intermediate elements may be present. In contrast, when an element is referred to as being "directly connected to" or "directly coupled to" another element, it should be understood that no intermediate elements are present. Expressions describing relationships between elements, such as "between," "immediately between," or "directly adjacent to," should be interpreted in the same manner.

The terminology used herein is for the purpose of describing particular embodiments only and is not intended to be limiting of the invention. Singular forms are intended to include plural forms unless the context clearly indicates otherwise. In this specification, terms such as "comprise" or "have" specify the presence of stated features, numbers, steps, operations, elements, components, or combinations thereof, but do not preclude the presence or addition of one or more other features, numbers, steps, operations, elements, components, or combinations thereof.

Unless otherwise defined, all terms used herein, including technical and scientific terms, have the same meaning as commonly understood by one of ordinary skill in the art to which this invention belongs. Terms defined in generally used dictionaries should be interpreted as having the meaning consistent with the context of the related art, and should not be interpreted in an idealized or overly formal sense unless explicitly defined in this specification.

Hereinafter, embodiments will be described in detail with reference to the accompanying drawings. However, the scope of the patent application is not limited or restricted by such embodiments. The same reference numerals used in each drawing denote the same elements.

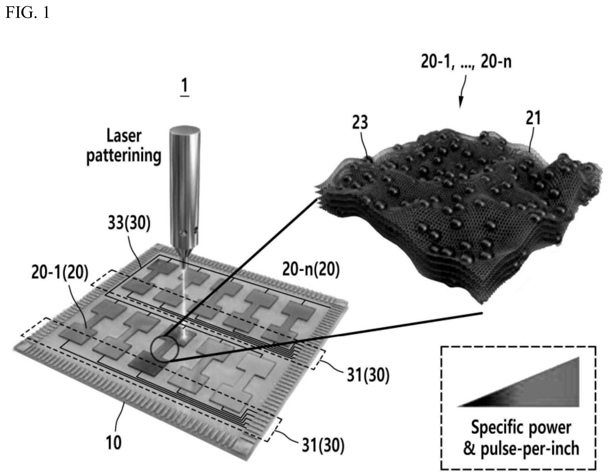

FIG. 1 is a configuration diagram of an electronic nose (1) including an olfactory sensor array (20) comprising a porous laser-induced graphene layer (21) in which gas-active particles (23) are embedded, according to an embodiment.

Referring to FIG. 1, the electronic nose (1) according to an embodiment may comprise a substrate (10), an olfactory sensor array (20), and contact pads (30). The olfactory sensor array (20) may comprise a plurality of olfactory sensors (20-1 to 20-n).

The substrate (10) may comprise a carbon precursor and a gas-active material precursor. Specifically, the substrate (10) may be formed using a mixture comprising the carbon precursor and the gas-active material precursor.

The carbon precursor may be a polymer containing carbon, and may form the laser-induced graphene layer (21) by laser irradiation described below. The laser-induced graphene layer (21) may comprise a plurality of graphene layers and may have a porous structure formed by irradiating the carbon precursor with a laser. The laser-induced graphene layer (21) may have a large surface area and abundant surface functional groups, thereby being able to bind with the gas-active particles and form olfactory sensors having high gas detection sensitivity. An odor substance may react with the laser-induced graphene layer (21), thereby generating an electrical signal.

The carbon precursor may comprise polydimethylsiloxane (PDMS), polyimide (PI), polyurethane (PU), cellulose, cellulose acetate, acetate butyrate, cellulose acetate propionate, polymethyl methacrylate (PMMA), polymethyl acrylate (PMA), polyacrylic copolymers, polyvinyl acetate copolymers, polyvinyl acetate (PVAc), polyvinylpyrrolidone (PVP), polyvinyl alcohol (PVA), polyfurfuryl alcohol (PPFA), polystyrene (PS), polystyrene copolymers, polyethylene oxide (PEO), polypropylene oxide (PPO), polyethylene oxide copolymers, polypropylene oxide copolymers, polycarbonate (PC), polyvinyl chloride (PVC), polycaprolactone, polyvinyl fluoride, polyvinylidene fluoride copolymers, polyamide, polyethylene terephthalate (PET), polyethylene (PE), or a mixture thereof. In particular, the carbon precursor may be polyimide.

The gas-active material precursor may form gas-active particles by laser irradiation. The gas-active particles may refer to metal oxides serving as redox catalysts that promote reaction with gaseous substances, i.e., odor substances. The gas-active material precursor may comprise components for generating the gas-active particles by laser irradiation.

The gas-active particles may comprise cerium oxide ($CeO_2$), zinc oxide (ZnO), tin oxide ($SnO_2$), indium oxide ($In_2O_3$), zirconium oxide ($ZrO_2$), tungsten oxide ($WO_3$), iron oxide ($Fe_2O_3$), copper oxide (CuO), or a mixture thereof.

The olfactory sensor array (20) may comprise a plurality of olfactory sensors (20-1 to 20-n) formed on the substrate (10). The olfactory sensors (20-1 to 20-n) may function to generate and transmit signals when exposed to gases.

The olfactory sensors (20-1 to 20-n) may be formed by irradiating lasers having different parameters onto the substrate comprising the carbon precursor mixed with the gas-active material precursor, so that they respond to different gaseous substances (odor substances). The olfactory sensors (20-1 to 20-n) may be individually formed by irradiating lasers onto specific regions distinguishable from each other on the substrate. When the substrate is irradiated with a laser, the substrate may form the laser-induced graphene layer (21) because it comprises the carbon precursor. The gas-active particles may be embedded in the laser-induced graphene layer (21).

The olfactory sensors (20-1 to 20-n) formed by irradiating lasers having different parameters may have different physical and chemical properties, and thus may be formed to respond to different odor substances. Accordingly, this may enable olfactory combination coding for odor molecule classification.

In the olfactory sensor constituting the electronic nose according to an embodiment, the gas-active particles (23) may act as an n-type semiconductor, and the laser-induced graphene layer (21) may act as a p-type semiconductor to form a heterojunction. In this case, the gas-active particles (23) may be embedded in the form of nanometer-sized particles on the laser-induced graphene layer (21).

In the electronic nose according to an embodiment, the contact pads (30) may comprise wirings that electrically connect the olfactory sensors (20-1 to 20-n) on the upper portion of the substrate (10) so as to output resistance changes of the olfactory sensors (20-1 to 20-n) according to redox reactions caused by gaseous substances.

The wirings may comprise signal lines (31) configured to output resistance changes of the olfactory sensors (20-1 to 20-n) according to redox reactions caused by gaseous substances, and ground lines (33) configured to commonly ground the olfactory sensors (20-1 to 20-n).

The electronic nose (1) may further comprise an odor prediction model trained by an artificial intelligence algorithm to analyze signal output patterns of the plurality of olfactory sensors (20-1 to 20-n) and to predict types of gaseous substances, i.e., odor substances, to which the plurality of olfactory sensors respectively respond, as well as concentrations of the odor substances.

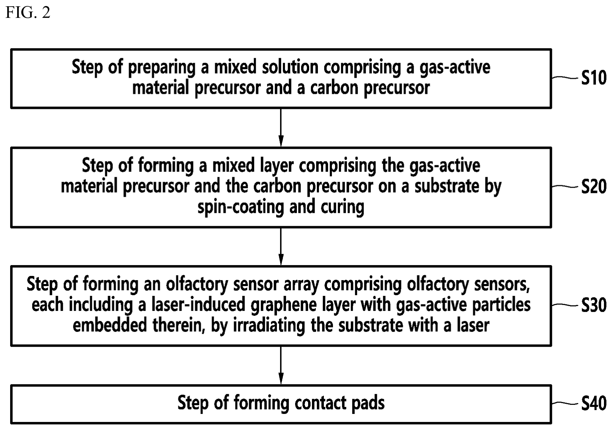

Meanwhile, FIG. 2 is a flowchart illustrating a method of manufacturing an electronic nose according to an embodiment.

Referring to FIG. 2, the method of manufacturing an electronic nose according to the embodiment may comprise: preparing a mixed solution (S10); forming a substrate (10) using the mixed solution (S20); generating an olfactory sensor array (20) including a plurality of olfactory sensors (20-1 to 20-n) (S30); and forming contact pads (30) (S40).

In the step of preparing the mixed solution (S10), a gas-active material precursor may be mixed with a solvent to form a gas-active material precursor solution. The solvent may comprise various conventional solvents used to dissolve a metal precursor. For example, the solvent may comprise at least one selected from the group consisting of 1-methyl-2-pyrrolidinone (NMP), gasoline, benzene, toluene, hexane, thinner, methylene chloride, ether, acetone, methyl ethyl ketone (MEK), methanol, ethanol, n-propanol, iso-propanol, n-butanol, isobutanol, t-butanol, and pentanol.

This step may further comprise dissolving a carbon precursor to form a liquid carbon precursor solution. The step of preparing the mixed solution comprising the gas-active material precursor and the carbon precursor (S 10) may comprise mixing and stirring the gas-active material precursor solution and the carbon precursor solution to form the mixed solution.

In the step of forming the substrate (S20) using the mixed solution, the mixed solution comprising the gas-active material precursor and the carbon precursor may be applied onto a base layer or a mold and then cured to form the substrate. The base layer may be a glass substrate or a silicon substrate. For example, in this step, the mixed solution may be spin-coated on the base layer, followed by soft baking, to form the substrate comprising the gas-active material precursor and the carbon precursor. In particular, this step may comprise performing at least one cycle including forming a mixed layer by spin-coating the mixed solution on the upper surface of the base layer and curing the mixed layer, thereby forming the substrate (10) on the base layer with a predetermined thickness.

The step of generating the olfactory sensor array (S30) may comprise irradiating lasers having different output characteristics onto the mixed layer comprising the gas-active material precursor and the carbon precursor, thereby forming an olfactory sensor array (20) comprising olfactory sensors (20-1 to 20-n) each including a laser-induced graphene layer (21) in which gas-active particles (23) are embedded and configured to respond to different gaseous substances.

In this step, the plurality of olfactory sensors may be individually formed by differently adjusting parameters of the laser, the parameters comprising at least one selected from the group consisting of a specific power of the laser, a pulse per inch (PPI) of the laser, and a laser scanning speed, such that the plurality of olfactory sensors respectively respond to different types of gaseous substances. When olfactory sensors are formed on the substrate by irradiating lasers having different parameters as described above, the olfactory sensors may have different physical and chemical properties, and therefore may be configured to respond to different odor substances.

The step of forming the contact pads (S40) may comprise forming contact pads (30) including signal lines (31) for signal output of the olfactory sensors (20-1 to 20-n) and ground lines (33). The contact pads (30) may be coated with a conductive paste so as to be stably connected to probe tips.

Hereinafter, the present invention will be described in further detail by way of examples. The examples presented are merely specific illustrations of the invention and are not intended to limit the technical scope of the invention.

<Example>

(1) Fabrication of Laser-Induced Graphene layer (LIG) Including CeOx Particles 720 mg of $Ce(NO_3)_3 \cdot 6H_2O$ was mixed with 8.3 ml of NMP (1-methyl-2-pyrrolidinone) and dissolved by ultrasonic treatment to prepare a cerium precursor solution. The cerium precursor solution was mixed with 41.7 ml of a carbon precursor solution, and the mixture was shaken at room temperature to prepare a mixed solution. The carbon precursor solution was prepared using liquid PI (polyimide), and a total of 50 ml of the mixed solution was prepared.

The mixed solution was spin-coated at 2000 rpm for 30 seconds onto a glass substrate covered with Kapton tape, followed by soft baking at 100 °C. The unit cycle including spin-coating and soft baking was repeated five times to form a substrate having a predetermined thickness, and final curing was performed at 200 °C.

Next, for fabricating cerium particle-embedded laser-induced graphene (CeLIG), a laser process was performed by fixing the laser scanning speed at 50% of the maximum scanning speed and adjusting the specific power and the pulse per inch (PPI). In this process, a 25 W laser cartridge was used, and the focal point was adjusted to the surface of the substrate.

For the fabrication of olfactory sensors, vector scanning was applied to pattern filament-type channels with a length of 1 mm. The contact pads were patterned by rastering and coated with Ag paste so as to be stably connected to probe tips.

Accordingly, a total of ten different CeLIG olfactory sensors (hereinafter referred to as "channels (Ch1 to Ch10)") were fabricated by varying the laser process parameters (Ch1: 5% specific power and 200 PPI, Ch2: 5% specific power and 500 PPI, Ch3: 10% specific power and 200 PPI, Ch4: 10% specific power and 500 PPI, Ch5: 15% specific power and 100 PPI, Ch6: 15% specific power and 200 PPI, Ch7: 15% specific power and 500 PPI, Ch8: 20% specific power and 100 PPI, Ch9: 20% specific power and 200 PPI, Ch10: 20% specific power and 500 PPI), thereby forming an olfactory sensor array.

<Experimental Example>

(1) Characterization

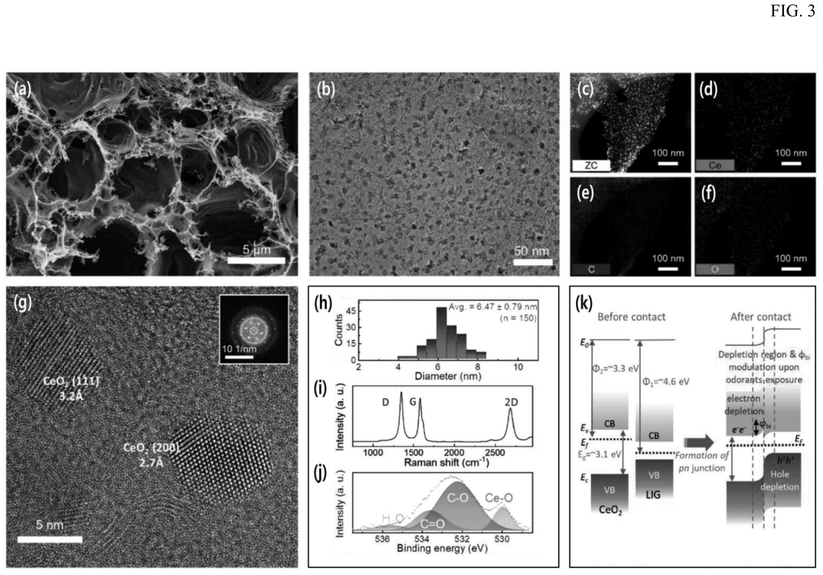

The characteristics of the CeLIG fabricated by the method according to the embodiment were analyzed, and the results are shown in FIG. 3. The characterization of CeLIG was performed using field emission scanning electron microscopy (FE-SEM), ultra-high-resolution transmission electron microscopy (TEM), high-resolution Raman spectroscopy, X-ray photoelectron spectroscopy (XPS), and ultraviolet photoelectron spectroscopy (UPS).

FIG. 3 shows analysis results of the characteristics of the CeLIG sensor formed by the method according to an embodiment.

Specifically, FIG. 3(a) is an SEM image of the CeLIG showing macroporous characteristics, FIG. 3(b) is a TEM image of the CeLIG, and FIGS. 3(c) to 3(f) are EDS-TEM images of the CeLIG showing the distribution maps of electrons, cerium, carbon, and oxygen, respectively. FIG. 3(g) is a high-magnification TEM image of the CeLIG showing the $CeO_2$ lattice structure. FIG. 3(h) shows the uniform size distribution of $CeO_x$ particles included in the LIG. FIG. 3(i) shows the Raman spectrum, FIG. 3(j) shows the XPS O1s spectrum of the CeLIG, and FIG. 3(k) is an energy band diagram of the CeLIG showing pn junction formation between typical $CeO_x$ and LIG.

As confirmed from the SEM image of FIG. 3(a), the CeLIG fabricated by the method according to the embodiment exhibited a typical interlaced porous structure of LIG. The formation of many macropores was presumed to result from local explosions and the release of gaseous species during photothermal decomposition of the polyimide film doped with cerium (Ce) under laser irradiation. Such pores are important for increasing surface area, which is advantageous for gas sensing applications.

As confirmed from the TEM image of FIG. 3(b) and the EDS images of FIGS. 3(c) to 3(f), $CeO_2$ particles were uniformly distributed throughout the laser-induced graphene layer in the CeLIG of the embodiment. As confirmed from the high-resolution TEM image of FIG. 3(g), characteristic lattice spacings of 0.32 nm and 0.27 nm corresponding to the (111) and (200) planes of $CeO_2$, respectively, were clearly observed. In particular, the average particle size of the $CeO_2$ particles was 6.47 $\pm$ 0.79 nm (FIG. 3(h), n=150), showing a very narrow size distribution and no signs of aggregation.

The homogeneity of the $CeO_2$ particles in the CeLIG was inferred from the rapid heating/cooling process during nucleation and the influence of the laser-induced graphene layer (LIG) acting as a functional backbone. The photothermal effect induced by laser irradiation was confirmed to provide optimal conditions for nanoparticle synthesis. The rapid heating caused simultaneous nucleation and growth in the irradiated region, resulting in a uniform nanoparticle size distribution.

During the thermal process induced by the laser, nanoparticle growth occurred while minimizing the movement of nanoclusters, and a defective backbone was formed for strong surface bonding with the nanoclusters.

As confirmed from FIG. 3(i), the laser-induced graphene layer (LIG) had abundant defect sites and functional groups, as indicated by a strong D peak in the Raman spectrum of graphite materials.

As confirmed from the XPS results in FIG. 3(j), the O1s spectrum showed a strong C-O peak (532.1 eV) and a C=O peak (533.5 eV), together with an O-Ce peak (529.9 eV). This indicated the presence of hydroxyl and carboxyl groups on the LIG carbon surface. Accordingly, the surface-functionalized LIG carbon backbone was considered to assist in stabilization and immobilization of cerium nanoclusters and to prevent aggregation. The $CeO_2$ particles formed in situ as a monolithic structure were confirmed to be more strongly bonded to the backbone compared with ex-situ delivery methods, providing enhanced stability for applications in gas sensing, catalytic reactions, or mechanical operations. Thus, it was confirmed that the laser process enabled uniform doping of small $CeO_2$ particles into the LIG.

As confirmed from the energy band diagram of FIG. 3(k), the gas-sensing performance of the CeLIG doped with $CeO_x$ could be verified. When n-type $CeO_2$ having a bandgap energy of 3.1 eV and a work function of 3.3 eV formed a junction with p-type narrow bandgap LIG having a work function of 4.5 eV, a depletion layer was formed due to the built-in potential, and the pn heterojunction at the interface was confirmed to enhance sensing performance of gas sensors based on metal oxides and various carbon derivatives. The heterostructure with well-dispersed particles maximized the number of depletion layers, thereby amplifying the conversion of chemical changes into electrical signals. When the adsorbed target gas was a reducing gas, the electron concentration of the CeOx increased, strengthening the pn junction, thereby widening the depletion layer and increasing the resistance. In contrast, when the adsorbed gas was an oxidizing gas, the energy band shifted in the opposite direction, resulting in decreased resistance. Therefore, even minor changes in charge carrier concentration could be effectively converted into significant sensor responses, thereby improving odor detection performance.

(2) Diversification of Chemical and Structural Properties of Olfactory Sensors by Varying Laser Parameters

An olfactory sensor array having different chemical and physical properties can generate distinguishable response patterns to various odor substances by applying a combination coding approach, thereby improving classification accuracy.

For this purpose, laser parameters such as specific power and pulse per inch (PPI) were adjusted. Here, the PPI parameter may refer to pulse stacking density along the laser scanning direction.

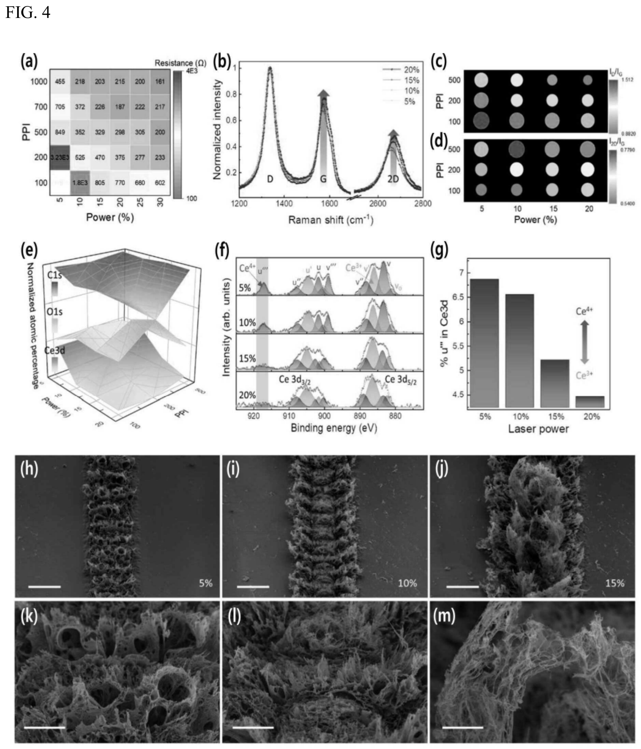

FIG. 4 shows analysis results of the chemical and physical diversity of the CeLIG sensor array formed by the method according to an embodiment.

Specifically, FIG. 4(a) is a heat map of electrical resistance with respect to the specific power and PPI of laser irradiation, FIG. 4(b) is a Raman spectrum of the CeLIG with different specific powers (fixed at 100 PPI), FIG. 4(c) is the ID/IG ratio, FIG. 4(d) is the I2D/IG ratio as a function of laser irradiation parameters, FIG. 4(e) shows normalized atomic percentages of carbon, oxygen, and cerium with variation of specific power and PPI of laser irradiation, FIG. 4(f) is an XPS Ce 3d spectrum with increasing specific power (fixed at 200 PPI), FIG. 4(g) shows the area percentage of the separated $u'''$ peaks indicating compositional variation of $Ce^{3+}$ and $Ce^{4+}$ with changing specific power (fixed at 200 PPI), and FIGS. 4(h) to 4(m) are SEM images of the CeLIG treated with various specific powers at fixed 200 PPI (H and K: 5%, I and L: 10%, J and M: 15%). The scale bars are 200 $\mu$m for H to J and 50 $\mu$m for K to M.

As confirmed from FIG. 4(a), evaluation of electrical resistance changes in response to various laser parameters showed that resistance decreased as the laser power and PPI increased, since higher laser fluence promoted graphitization of the PI substrate.

FIGS. 4(b) to 4(d) show Raman spectroscopy results characterizing carbon derivatives. The G and D peaks indicated that the polymer precursor was converted into an $sp^2$ carbon-rich material. The presence of the 2D peak, which is the second overtone of the D peak, served as a fingerprint for graphite materials. As confirmed from FIGS. 4(b) to 4(d), as the specific power of the laser increased from 5% to 20% and the PPI increased from 100 to 500, the ID/IG ratio representing defect density decreased from a maximum of 1.51 to a minimum of 0.88. Conversely, the I2D/IG ratio, which represents thinner $sp^2$ carbon layers and graphene quality, increased from a minimum of 0.54 to a maximum of 0.77. The increased laser fluence was predicted to induce local high-temperature and high-pressure conditions, enhancing carbonization, graphitization, and exfoliation, thereby generating thinner and higher-quality $sp^2$ carbon layers. These results were consistent with the resistance heat map results (see FIG. 4(a)).

Through the embodiment, it was confirmed that various sensors having different response characteristics depending on the degree of graphene exfoliation, surface oxidation, and defect density could be fabricated simply by changing laser irradiation conditions (i.e., parameters) without complex wet chemistry.

FIG. 4(e) is a three-dimensional mapping graph of atomic percentages of carbon, oxygen, and cerium calculated using XPS surface analysis, with each element normalized for visualization. It was confirmed that in the CeLIG according to the embodiment, when irradiated with a laser under room-temperature conditions, the carbon surface was oxidized as the specific power increased. As a result, the carbon content decreased while the oxygen content increased. This change was most pronounced under the condition of 200 PPI. In addition, the atomic percentage of cerium varied from 0.19% to 0.89% depending on the laser parameters.

The variation of laser parameters was confirmed to affect not only the carbon backbone of the CeLIG but also the diversity of CeOx particles. For this purpose, the chemical environment and valence states of the CeOx catalytic particles in the CeLIG were analyzed through XPS Ce 3d scans, and the results are shown in FIG. 4(f). Specifically, the oxidation states of cerium significantly influenced energy structure, bandgap, and redox catalytic activity, thereby diversifying the gas response patterns.

Specifically, the amount of $Ce^{3+}$ can directly enhance the catalytic activity of cerium oxide. The Ce 3d core-level spectrum was divided into "u" and "v" groups corresponding to Ce $3d_{3/2}$ and Ce $3d_{5/2}$, respectively, with a spin-orbit splitting of 18.6 eV. Each component was further divided into multiple peaks. Peaks v (882.8 eV), v" (887.8 eV), $v'''$ (898.6 eV), u (901.4 eV), u" (907.4 eV), and $u'''$ (916.9 eV) were attributed to the $Ce^{4+}$ state, whereas peaks $v_0$ (880.5 eV), v' (885.4 eV), $u_0$ (900.1 eV), and u' (904.2 eV) represented the $Ce^{3+}$ state. This indicated coexistence of mixed valence states Ce(III) and Ce(IV) within CeOx, implying high redox activity of cerium. Quantification of $Ce^{3+}$ was implemen-

ted by calculating the relative area percentage of the u‴ peak, which is not related to the amount of $Ce^{3+}$ (the area of the u‴ peak is inversely proportional to the amount of $Ce^{3+}$). Accordingly, the proportion of $Ce^{3+}$ was confirmed to increase as the laser power increased (see FIG. 4(g)). This phenomenon was considered to result from oxygen vacancies generated in the metal oxide at high temperatures due to increased laser power, thereby promoting the formation of $Ce^{3+}$ to maintain charge neutrality. Thus, it was determined that changing laser parameters created a gradient in the amount of $Ce^{3+}$, and consequently, the catalytic activity of CeOx could be tuned.

Furthermore, as the amount of $Ce^{3+}$ increased, the Ce 4f orbitals became partially filled, narrowing the bandgap and altering the depletion region (see FIG. 3(k)). Through such changes, it was confirmed that gas response patterns could be diversified, enabling the design of the sensor array.

In addition, the laser process was also confirmed to impart morphological diversity to the LIG.

As confirmed from the SEM images of FIGS. 4(h) to 4(m), the formation of a large number of anisotropic fibers was further promoted as the specific power increased. However, under low-power conditions, an isotropic porous structure was generated. It was confirmed that rapid gas generation induced by laser irradiation sharply increased local pressure, simultaneously causing carbonization, expansion, and exfoliation, thereby converting the LIG from a porous structure into a fiber-like structure.

(3) Classification of Odor Substances Using the CeLIG-Based Electronic Nose

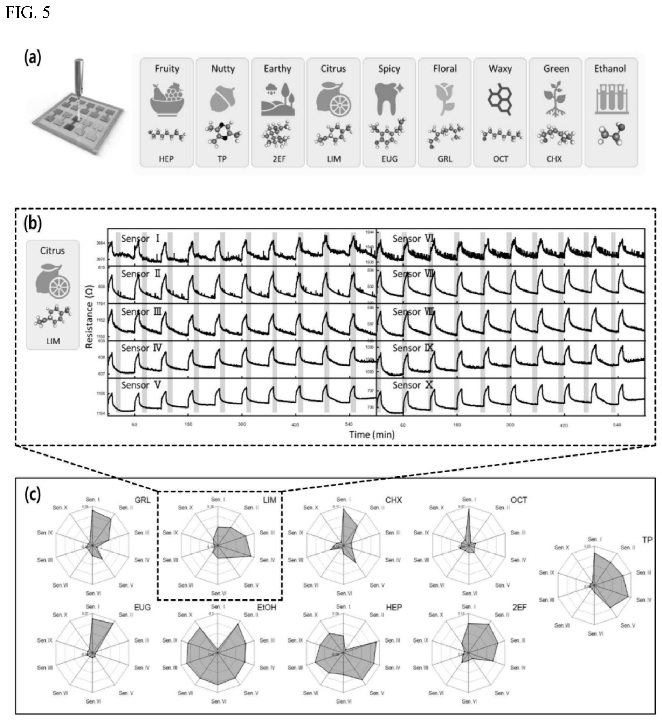

FIG. 5 shows response data of the CeLIG sensor array formed by the method according to an embodiment with respect to different odor substances.

Specifically, FIG. 5(a) illustrates structural formulas of nine odor substance molecules used in the fragrance industry (1-heptanol, 2,3,5-trimethylpyrazine, 2-ethylphenol, d-limonene, eugenol, geraniol, octanal, cis-3-hexenol, and ethanol). FIG. 5(b) shows dynamic sensing curves of ten CeLIG olfactory sensors with respect to d-limonene under a gas flow of 1000 sccm (10 min exposure, 50 min recovery). FIG. 5(c) is a polar coordinate plot showing different response patterns of ten olfactory sensors with respect to various odor substances. All measurements were conducted at room temperature without using a heater.

An artificial olfactory sensor array was fabricated by utilizing the chemical and physical diversity introduced into the CeLIG through variation of laser parameters (specific power and PPI).

The fabricated olfactory sensor array was tested against nine representative odor substances-geraniol (floral), d-limonene (citrus), octanal (waxy), eugenol (spicy), 1-heptanol (fruity), 2-ethylphenol (earthy), cis-3-hexenol (green), 2,3,5-trimethylpyrazine (nutty), and ethanol (alcoholic) (see FIG. 5(a)). These fragrances are non-toxic and have distinctive aromas, and thus are widely used in perfumes, food additives, and fragrances.

As shown in FIG. 5(b), when repeatedly exposed to these fragrances, the resistance values of each sensor changed, from which response values were calculated. Accordingly, ten features for each fragrance could be extracted from the sensor array.

The response values of the olfactory sensors were derived by applying Equation 1 below.

[Equation 1]

$$\text{Reactivity} = (R - R_0) / R_0 \times 100 \ (\%)$$

where R is the resistance of the olfactory sensor after exposure to the target gas, and $R_0$ is the initial resistance value of the olfactory sensor.

As confirmed from the radar graph shown in FIG. 5(c), the average response values could be determined. As a result, unique response patterns that could clearly distinguish fragrances were identified. In addition, unlike conventional metal oxide semiconductor gas sensors that generally require high temperatures (> 250 °C) or light sources for activation, the LIG-based gas sensor was confirmed to operate at room temperature, thereby providing high energy efficiency, simplified device configuration, and applicability to flexible substrates.

Odor molecules often possess multiple functional groups or complex structures. For example, such molecules may comprise various combinations of alkyl groups (methyl, ethyl, heptyl, etc.) of different lengths, which act as nearly nonpolar electron donors. Hydroxyl groups act as strong electron donors, phenol and ether groups act as weak donors, aldehydes act as weak acceptors, and pyrazine rings act as moderate acceptors. Consequently, such molecules have rarely been used in previous gas sensor studies to avoid complexity, and the response mechanisms remain unexplored.

In practical applications for the classification of various odor molecules, it may be inefficient to match the response mechanisms of gas sensor arrays with odor molecules in a one-to-one manner. Therefore, in the electronic nose according to the embodiment, a method was provided in which odor substances, i.e., gaseous substances, could be distinguished by utilizing response patterns of multiple olfactory sensors rather than one-to-one response mechanisms between individual olfactory sensors and odor molecules.

(4) Machine Learning-Based Data Processing

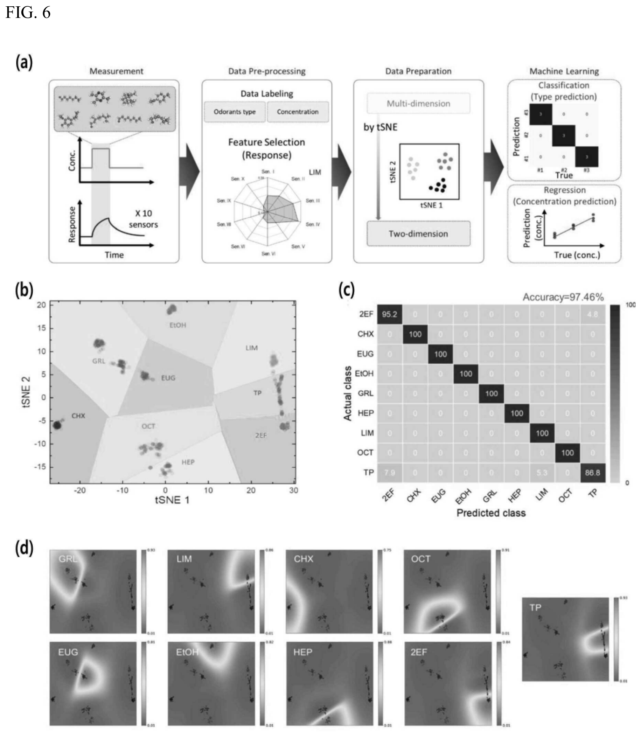

FIG. 6 illustrates an odor classification training process based on a machine learning algorithm using the CeLIG sensor array according to an embodiment.

Specifically, referring to FIG. 6, the response values of each olfactory sensor were used as features for data analysis. A t-distributed stochastic neighbor embedding (t-SNE) analysis was performed using the Python scikit-learn library to reduce multidimensional parameters into two dimensions. Classification of odor substance types and regression for concentration prediction using a support vector machine (SVM) were performed with the RBF kernel by the Python scikit-learn library. The dataset was divided into training and testing sets at a ratio of 7:3. The Tanimoto coefficient for comparing molecular similarity was calculated by converting each compound into a simplified molecular-input line-entry system (SMILES) notation and using the Python Rdkit library.

FIG. 6(a) illustrates the data processing and training procedure for machine learning-based odor prediction. As shown in FIG. 6(a), first, as indicated in FIG. 5, responses of ten integrated sensors to gas species of various types and conditions were collected. Then, classes and concentrations of odor molecules were assigned as labels to the dataset. For the machine learning feature data, response values of the ten sensors were extracted, and dimensionality reduction was performed using t-SNE to transform the data into a two-dimensional space. Through this unsupervised learning-based data preparation, high-dimensional data with many features could be represented in a reduced dimensional space while maintaining distances among data points, thereby being useful for computational efficiency and visualization. Finally, the labeled data (answer data) and feature data were used to train an SVM algorithm to generate an odor prediction model. The trained odor prediction model was utilized to classify types of odor substances and to predict their concentrations through regression.

The scatter plot of FIG. 6(b) represents the t-SNE results derived from the response values obtained from the integrated CeLIG olfactory sensor array. The t-SNE algorithm identified a two-dimensional representation that best preserved the distances among data points. Through manifold learning, the ten-dimensional Euclidean distance was transformed into two-dimensional coordinates, enabling each odor molecule class to be distinctly clustered without overlap. This approach provided meaningful visualization of large-scale datasets, which were otherwise complex to analyze in their original form or through radial plots (see FIG. 5(c)).

To identify and predict nine different aligned molecules, the obtained t-SNE data were used as feature data for machine learning training. Support vector machine (SVM), a widely used supervised learning technique for classification problems, is an algorithm that finds a hyperplane for classifying data and learns an effective classifier in the feature space using kernel methods. In the embodiment, the SVM model was implemented with a radial basis function (RBF) kernel to enhance the capability of handling nonlinear relationships in the data. The dataset was split into 70% for training and 30% for testing, the SVM model was trained on the training set, and its performance was evaluated on the testing set. For accurate generalization, the testing set was not used for parameter selection or model training.

FIG. 6(b) is a graph showing the decision boundary map of the two-dimensional t-SNE coordinates obtained by applying the SVM model. As confirmed from FIG. 6(b), the trained model provided distinguishable boundaries for nine odor substances within the reduced-dimensional space, thereby delivering clear visual information.

FIG. 6(c) is a graph showing a confusion matrix of actual classes and predicted classes for nine odor substances from the trained SVM classification results. As confirmed from FIG. 6(c), the trained model exhibited high prediction accuracy of 97.46% in the training set and 97.06% in the testing set, confirming successful generalization without overfitting or underfitting.

FIG. 6(d) is a diagram showing uncertainty estimates of the classifier in the form of probability prediction. In FIG. 6(d), each subplot corresponds to a specific odor substance class, and the color gradient of the heat map represents the confidence level of the model in predicting each class. Narrow and separated regions on the map indicated high probability, representing high classification confidence of the classifier. In contrast, wide regions indicated low probability, representing uncertain or low-confidence areas of classification.

Accordingly, as confirmed from FIG. 6(d), the trained classifier exhibited concentrated high-probability regions with minimal overlap, confirming a low risk of false positives or false negatives.

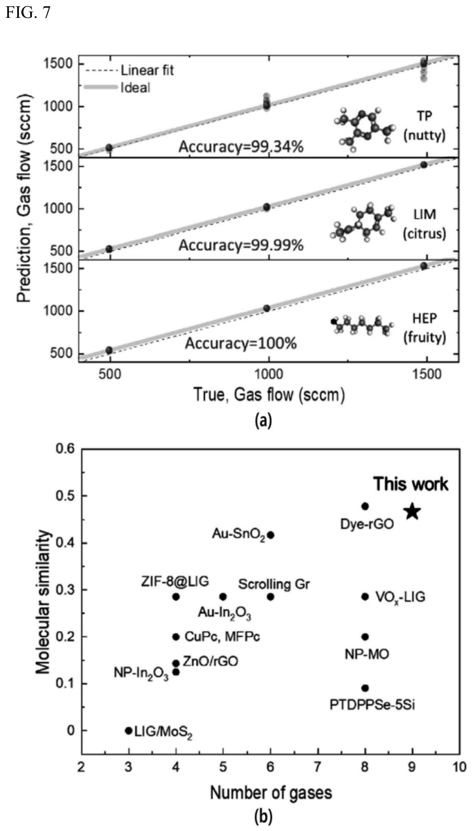

FIG. 7 shows odor prediction data and performance comparison results based on a machine learning algorithm using the CeLIG sensor array according to an embodiment. In FIG. 7, the concentrations of odor substances were predicted using support vector regression (SVR) of the scikit-learn machine learning library. The concentration of a fragrance plays an important role in determining its intensity, persistence, and overall perception.

FIG. 7(a) plots actual concentration data on the x-axis and predicted data on the y-axis as the ratio of carrier gas to base gas increased from 1:3, 1:1, to 3:1. The solid line represents the ideal prediction curve ($y = x$), and the dashed line represents a linear approximation of the predicted data using regression. The $R^2$ accuracy calculated using residual variance for the testing set was also high: 96.34% for 2,3,5-trimethylpyrazine, 94.44% for d-limonene, and 99.92% for 1-heptanol (99.34%, 99.99%, and 100% for the training set, respectively). These results indicated that t-SNE-based data processing provided clear and intuitive visualization, and that the machine learning-based model was capable of accurately predicting both the type and concentration of odor substances.

To quantitatively evaluate the selectivity of the fabricated olfactory sensor array, comparisons were made with previously reported chemiresistive gas sensors operating at room temperature. Among various computational methods, the Tanimoto coefficient was selected to measure molecular similarity. FIG. 7(b) shows the number of gas types used for selectivity evaluation of reported chemiresistive gas sensors and molecular similarity (including those studies). The molecular similarity used here was derived by calculating the maximum Tanimoto coefficient for all possible gas pairs. According to the results, the fabricated CeLIG olfactory sensor array was able to effectively discriminate multiple types of similar gases (odors). This comparison included not only electronic noses designed for odor discrimination but also single sensors targeting specific gases.

As confirmed from FIG. 7(b), an artificial olfactory platform capable of distinguishing a large number of odor molecules at room temperature, combined with structural simplicity, low energy consumption, and stability, could be applied to extend the artificial olfactory platform to a wide range of applications beyond conventional targets such as NO, $NH_3$, and specific VOCs, to include more complex odor molecules.

(5) Application to Flexible Devices

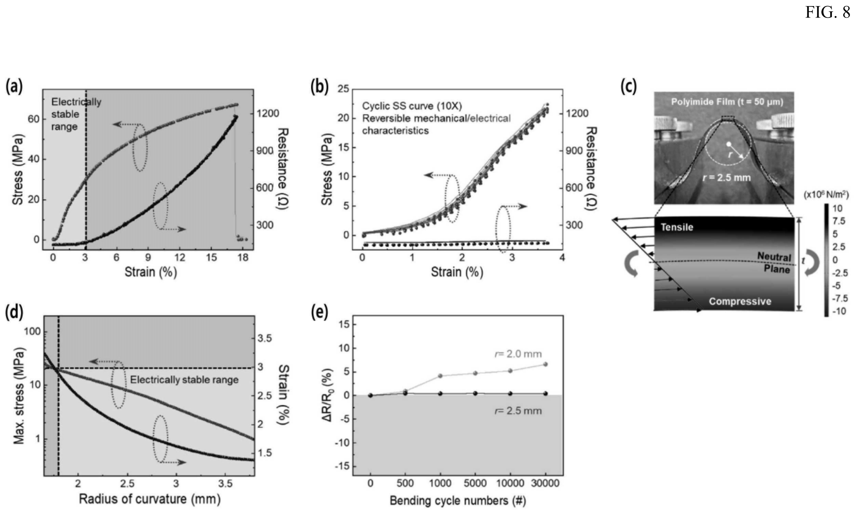

FIG. 8 shows stress analysis and flexibility test results of the CeLIG.

Flexibility is an important characteristic for applying sensor platforms in various fields such as patch-type sensors, smart packaging, or outdoor environments. Even when excellent mechanical stability is achieved, unstable electrical properties may interfere with sensor operation. To ensure reliable performance, it is important to establish an operating range in which both mechanical and electrical stability are maintained.

Referring to FIG. 8(a), the operating range for measuring mechanical stress and electrical resistance variation during tensile processes should be limited within a strain of 3% (indicated as the blue-shaded area), in which both mechanical and electrical stability are ensured.

In addition, in the case of the superelastic flexible substrate used in the embodiment, it was confirmed that when a load lower than the previously applied maximum load was applied, nonlinear and irreversible elastic behavior occurred due to stress softening known as the Mullins effect. To more accurately predict and analyze mechanical behavior, cyclic loading and unloading tests within a specific strain range were performed to observe material behavior, which needed to be taken into account. For this purpose, as shown in FIG. 8(b), repeated stress-strain (SS) characteristics were measured within the previously set strain range of 3%. Then, in order to improve prediction accuracy, the conditions and results minimizing variations in SS characteristics were applied to the mechanical behavior analysis.

Based on the data of FIG. 8(b), finite element analysis (FEA) results were used to visualize the spatial distribution of internal stress during bending and to identify the location of maximum stress (see FIG. 8(c)).

Through the analysis, as shown in FIG. 8(d), the achievable minimum bending radius was predicted. The maximum stress and maximum strain occurred on the outermost surface of the flexible structure, and bending occurred at the smallest curvature radius during bending. As shown in FIG. 8(d), the flexible structure could be designed to operate within an electrically and mechanically stable deformation range (electrically stable range, bending radius > 1.7 mm).

However, the results obtained from this FEA analysis did not take into account fatigue failure that may initiate from invisible microcracks as cyclic loading progresses. Therefore, direct verification through cyclic loading tests was required. Based on the FEA results incorporating the Mullins effect, fatigue tests were performed by selecting small bending radii of 2.0 mm and 2.5 mm, which fall within the electrically and mechanically stable range (see FIG. 8(e)). Although the bending radius of 2.0 mm was within the stable range, the structure maintained operational stability only for a limited number of bending cycles during the cyclic fatigue test. However, as the number of cycles exceeded 1000, invisible damage accumulated and propagated, impairing structural stability. In contrast, at the bending radius of 2.5 mm, which provided a safer margin within the electrically stable range, the initial resistance value was preserved even after 30,000 cycles of cyclic bending.

These results indicated that through flexibility optimization, the device could operate stably under repeated bending conditions without fatigue failure. Therefore, it was confirmed that the CeLIG platform could be applied to future applications requiring a flexible electronic nose.

As described above, although the embodiments have been explained with reference to limited drawings, it will be understood by those skilled in the art that various modifications and variations can be made from the foregoing disclosure. For example, the described techniques may be performed in an order different from that described, and/or the components of the described systems, structures, devices, or circuits may be combined or arranged in different forms, or replaced or substituted with other components or equivalents, while still achieving appropriate results.

Therefore, other implementations, other embodiments, and equivalents to the claims are also within the scope of the claims set forth below.

**Claims**

1.   An electronic nose comprising:

        a substrate comprising a carbon precursor and a gas-active material precursor; and
        an olfactory sensor array comprising a plurality of olfactory sensors formed by respectively irradiating the substrate with lasers having different parameters,
wherein each of the olfactory sensors comprises a laser-induced graphene layer formed from the carbon precursor and gas-active particles formed from the gas-active material precursor and embedded in the laser-induced graphene layer by laser irradiation with a single parameter, wherein the plurality of olfactory sensors generates a distinct response pattern for each type of gaseous substance, enabling their discrimination.

2. The electronic nose of claim 1, wherein parameters each comprise at least one selected from specific power of the laser, pulse per inch (PPI) of the laser, and laser scanning speed.

3. The electronic nose of claim 1 or 2, wherein in the olfactory sensors, the gas-active particles function as n-type semiconductors and the laser-induced graphene layer functions as a p-type semiconductor to form a heterojunction structure.

4. The electronic nose of one of claims 1 to 3, wherein the gas-active particles comprise at least one selected from the group consisting of cerium oxide ($CeO_2$), zinc oxide (ZnO), tin oxide ($SnO_2$), indium oxide ($In_2O_3$), zirconium oxide ($ZrO_2$), tungsten oxide ($WO_3$), iron oxide ($Fe_2O_3$), and copper oxide (CuO).

5. The electronic nose of one of claims 1 to 4, wherein the carbon precursor comprises at least one selected from the group consisting of polydimethylsiloxane (PDMS), polyimide (PI), polyurethane (PU), cellulose, cellulose acetate, acetate butyrate, cellulose acetate propionate, polymethyl methacrylate (PMMA), polymethyl acrylate (PMA), polyacrylic copolymer, polyvinyl acetate copolymer, polyvinyl acetate (PVAc), polyvinylpyrrolidone (PVP), polyvinyl alcohol (PVA), polyfurfuryl alcohol (PPFA), polystyrene (PS), polystyrene copolymer, polyethylene oxide (PEO), polypropylene oxide (PPO), polyethylene oxide copolymer, polypropylene oxide copolymer, polycarbonate (PC), polyvinyl chloride (PVC), polycaprolactone, polyvinyl fluoride, polyvinylidene fluoride copolymer, polyamide, polyethylene terephthalate (PET), and polyethylene (PE).

6. The electronic nose of one of claims 1 to 5, further comprising contact pads including wiring respectively connected to the plurality of olfactory sensors to output resistance corresponding to gas activation of the olfactory sensor array.

7. The electronic nose of one of claims 1 to 6, further comprising an odor prediction model trained by an artificial intelligence algorithm to predict gaseous substances to which each of the plurality of olfactory sensors responds and concentrations of the gaseous substances by analyzing signal output patterns of each of the plurality of olfactory sensors.

8. A method of manufacturing an electronic nose, comprising:

preparing a mixed solution comprising a gas-active material precursor and a carbon precursor;
forming a substrate using the mixed solution; and
generating an olfactory sensor array comprising a plurality of olfactory sensors by respectively irradiating the substrate with lasers having different parameters,
wherein each olfactory sensor comprises a laser-induced graphene layer formed from the carbon precursor and gas-active particles formed from the gas-active material precursor and embedded in the laser-induced graphene layer by laser irradiation with a single parameter, and
wherein the plurality of olfactory sensors generates a distinct response pattern for each type of gaseous substance, enabling their discrimination.

9. The method of claim 8, wherein the step of forming the substrate using the mixed solution comprises performing at least one unit cycle including spin-coating the mixed solution on an upper surface of a base layer to form a mixed layer and curing the mixed layer to form the substrate having a predetermined thickness.

10. The method of claim 8 or 9, wherein the step of generating the olfactory sensor array comprises forming each of the plurality of olfactory sensors by differentially controlling the parameters including at least one of specific power of the laser, pulse per inch (PPI) of the laser, and laser scanning speed so that the plurality of olfactory sensors respectively respond to different types of gaseous substances.

11. The method of one of claims 8 to 10, wherein the step of generating the olfactory sensor array further comprises forming contact pads for signal output of the olfactory sensors after generating the olfactory sensor array comprising the plurality of olfactory sensors.

FIG. 1

EP 4 764 482 A1

FIG. 2

| Step of preparing a mixed solution comprising a gas-active material precursor and a carbon precursor | S10 |

| Step of forming a mixed layer comprising the gas-active material precursor and the carbon precursor on a substrate by spin-coating and curing | S20 |

| Step of forming an olfactory sensor array comprising olfactory sensors, each including a laser-induced graphene layer with gas-active particles embedded therein, by irradiating the substrate with a laser | S30 |

| Step of forming contact pads | S40 |

13

FIG. 3

FIG. 4

FIG. 5

FIG. 6

FIG. 7

(a)

(b)

FIG. 8

Europäisches
Patentamt
European
Patent Office
Office européen
des brevets

# EUROPEAN SEARCH REPORT

**Application Number**

EP 25 21 2091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 113 758 975 A (UNIV EAST CHINA NORMAL) 7 December 2021 (2021-12-07) * fig. 1,2 and corresponding text passages * * claim 1 * | 1-11 | INV. G01N27/12 G01N33/00 |
| A | CN 117 142 535 B (UNIV TIANJIN) 3 September 2024 (2024-09-03) * claim 1 * * [0020] * | 1-11 | |
| A | YANG LI ET AL: "Novel gas sensing platform based on a stretchable laser-induced graphene pattern with self-heating capabilities", JOURNAL OF MATERIALS CHEMISTRY A, vol. 8, no. 14, 8 January 2020 (2020-01-08), pages 6487-6500, XP093368286, GB ISSN: 2050-7488, DOI: 10.1039/C9TA07855J * the whole document * | 1 | |
| A | ERIC SINGH ET AL: "Flexible Graphene-Based Wearable Gas and Chemical Sensors", ACS APPLIED MATERIALS & INTERFACES, vol. 9, no. 40, 29 September 2017 (2017-09-29), pages 34544-34586, XP055683931, United States ISSN: 1944-8244, DOI: 10.1021/acsami.7b07063 * p. 34569, right column, second par. * | 7 | |

**TECHNICAL FIELDS SEARCHED (IPC)**

G01N

The present search report has been drawn up for all claims

| Place of search | Date of completion of the search | Examiner |
|---|---|---|
| Munich | 24 February 2026 | Klein, Marc-Oliver |

## ANNEX TO THE EUROPEAN SEARCH REPORT
## ON EUROPEAN PATENT APPLICATION NO.

EP 25 21 2091

This annex lists the patent family members relating to the patent documents cited in  the above-mentioned European search report.
The members are as contained in the European Patent Office EDP file on
The European Patent Office is in no way liable for these particulars which are merely given for the purpose of information.

24-02-2026

| Patent document cited in search report | | Publication date | Patent family member(s) | Publication date |
|---|---|---|---|---|
| CN 113758975 | A | 07-12-2021 | NONE | |
| CN 117142535 | B | 03-09-2024 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240193844
- KR 101852074